# EUROPEAN PATENT SPECIFICATION

(11) **EP 1 495 769 B1**
(45) Date of publication and mention of the grant of the patent: **27.02.2008**
(21) Application number: 03077205.7
(22) Date of filing: 11.07.2003
(51) Int. Cl.: A61K 47/48, A61K 47/26, A61K 31/711, A61K 48/00, C07K 14/72, C12Q 1/68

(54) **Mannose-6-phosphate receptor mediated gene transfer into muscle cells**
Mannose-6-Phosphat Rezeptor vermittelter Gentransfer zu Muskelzellen
Transfert de gènes aux cellules musculaires au moyens du récepteur du mannose-6-phosphate

(43) Date of publication of application: 12.01.2005
(73) Proprietor: LBR Medbiotech B.V., 2333 CC Leiden (NL)
(72) Inventor: Platenburg, Gerard Johannes, 2252 XR Voorschoten (NL)
(74) Representative: van Westenbrugge, Andries

(56) References cited:
- US-B1- 6 172 208
- BONFILS E ET AL: "DRUG TARGETING: SYNTHESIS AND ENDOCYTOSIS OF OLIGONUCLEOTIDE- NEOGLYCOPROTEIN CONJUGATES" NUCLEIC ACIDS RESEARCH, OXFORD UNIVERSITY PRESS, SURREY, GB, vol. 20, no. 17, 11 September 1992 (1992-09-11), pages 4621-4629, XP000461068 ISSN: 0305-1048
- DEUTEKOM VAN J C T ET AL: "Antisense-induced exon skipping restores dystrophin expression in DMD patient derived muscle cells" HUMAN MOLECULAR GENETICS, OXFORD UNIVERSITY PRESS, SURREY, GB, vol. 10, no. 15, 2001, pages 1547-1554, XP002250908 ISSN: 0964-6906
- AARTSMA-RUS A ET AL: "Targeted exon skipping as a potential gene correction therapy for Duchenne muscular dystrophy" NEUROMUSCULAR DISORDERS, PERGAMON PRESS, GB, vol. 12, no. SUPPL 1, October 2002 (2002-10), pages S71-S77, XP002250906 ISSN: 0960-8966
- WILTON S D ET AL: "Specific removal of the nonsense mutation from the mdx dystrophin mRNA using antisense oligonucleotides." NEUROMUSCULAR DISORDERS: NMD. ENGLAND JUL 1999, vol. 9, no. 5, July 1999 (1999-07), pages 330-338, XP002261983 ISSN: 0960-8966
- MANN C J ET AL: "ANTISENSE-INDUCED EXON SKIPPING AND SYNTHESIS OF DYSTROPHIN IN THE MDX MOUSE" PROCEEDINGS OF THE NATIONAL ACADEMY OF SCIENCES OF USA, NATIONAL ACADEMY OF SCIENCE. WASHINGTON, US, vol. 98, no. 1, 2 January 2001 (2001-01-02), pages 42-47, XP001160821 ISSN: 0027-8424
- DUNCKLEY M G ET AL: "MODIFICATION OF SPLICING IN THE DYSTROPHIN GENE IN CULTURED MDX MUSCLE CELLS BY ANTISENSE OLIGONUCLEOTIDES" HUMAN MOLECULAR GENETICS, OXFORD UNIVERSITY PRESS, SURREY, GB, vol. 5, no. 1, July 1995 (1995-07), pages 1083-1090, XP000939302 ISSN: 0964-6906
- REUSER A J J ET AL: "UPTAKE AND STABILITY OF HUMAN AND BOVINE ACID ALPHA GLUCOSIDASE EC-3.2.1.20 IN CULTURED FIBROBLASTS AND SKELETAL MUSCLE CELLS FROM GLYCOGENOSIS TYPE II PATIENTS" EXPERIMENTAL CELL RESEARCH, vol. 155, no. 1, 1984, pages 178-189, XP009021228 ISSN: 0014-4827
- MARTINIUK FRANK ET AL: "Correction of glycogen storage disease type II by enzyme replacement with a recombinant human acid maltase produced by over-expression in a CHO-DHFRneg cell line" BIOCHEMICAL AND BIOPHYSICAL RESEARCH COMMUNICATIONS, vol. 276, no. 3, 5 October 2000 (2000-10-05), pages 917-923, XP002262096 ISSN: 0006-291X

## Description

### FIELD OF INVENTION

The present invention is in the field of glycoside conjugates. It relates to improve muscle uptake of compounds in general. In particular it relates to glycoside-oligonucleotide conjugates for use in antisense strategies, particularly in vivo antisense strategies.

### BACKGROUND OF THE INVENTION

A potential genetic therapy was explored, aimed at restoring the reading frame in muscle cells from Duchenne muscular dystrophy (DMD) patients through targeted modulation of dystrophin pre-mRNA splicing. Considering that exon 45 is the single most frequently deleted exon in DMD, whereas exon (45+ 46) deletions cause only a mild form of Becker muscular dystrophy (BMD), an antisense-based system was set up to induce exon 46 skipping from the transcript in cultured myotubes of mouse and of human origin. In myotube cultures from two unrelated DMD patients carrying an exon 45 deletion, the induced skipping of exon 46 in only 15% of the mRNA led to normal amounts of properly localized dystrophin (of course lacking the domains corresponding to exon 45 & 46) in at least 75% of myotubes (van Deutekom et al. 2001). Using the same antisense-based strategy using a different antisense sequence, in another study the skipping of 11 other exons was demonstrated in the dystrophin gene in cultured human myotubes (Aartsma-Rus et al. 2002). Technology to induce skipping of these 12 different exons would (in the population of DMD causing genetic defects), in total, allow correction of more than 50% of the deletions and 22% of the duplications in the population present in the Leiden DMD-mutation Database.

However, the biggest hurdle to overcome is the poor *in vivo* muscle uptake of these antisense oligonucleotides, and this applies for other molecules with therapeutic potential as well, by the relevant cells. An efficient therapy for DMD will require that essentially all of the skeletal muscles including those of arms and legs and the muscles involved in respiration as well as the cardiac muscle are targeted. None of the mechanisms investigated to date have the ability to specifically deliver (antisense) oligonucleotides, let alone entire genes, to essentially all muscle tissues/cells simultaneously over the entire body. Methods for the *in vivo* delivery of genes or other compounds into muscle that have been published so far include injection of naked DNA with or without electrotransfer, intravascular delivery (both reviewed in Herweijer and Wolff, 2003) and use of microbubbles (Lu et al. 2003). Direct injection of DNA into the skeletal muscle is a safe and simple method, but is hampered by low transfection efficiencies. The efficiencies can be significantly improved by pretreatment of the muscle with hyaluronidase followed by electrotransfer and using this method a dystrophin plasmid was expressed in 22% of the fibres in the muscle of an mdx mouse for up to 8 weeks (Gollins et al. 2003). A severe obstacle to clinical application of this method however, is the muscle fibre damage induced by the powerful electric fields required to achieve efficient gene delivery. A way to limit the damage to the muscles, is injection into skeletal muscle of a mixture of naked DNA and microbubbles. It was found that the use of a commercially available albumin-coated octa-fluoropropane gas microbubble, Optison, improves transfection efficiency and this was associated with a significant decrease in muscle damage (Lu et al. 2003). However, the major disadvantage of direct injection into muscles remains, being that each muscle has to be treated separately, and thus treatment of the entire muscle mass of an individual by these methods is not feasible.

The intravascular delivery of DNA is a more attractive method, because a whole muscle group can be covered with a single injection. Intravascular delivery via a catheter to limb skeletal muscle groups, in combination with blocking blood flow with a blood pressure cuff, has successfully been performed in rabbits, dogs and rhesus monkeys (Herweijer and Wolff, 2003). In rhesus monkeys, transfection efficiencies ranging from less than 1% to more than 30% in different muscles in leg and arm have been observed (Zhang et al. 2001). Also, it is claimed that delivery is not limited to skeletal muscle, but that delivery is also in the cardiac muscle (Herweijer et al., 2000). However, whole-body treatment would still require multiple injections and furthermore, treatment of the respiratory muscles seems impossible with this method.

Ideally, whole-body muscle therapy would use single intravenous injections of a compound endowed with a cell specific targeting ability. Up to date, two molecules have been described that have potential for muscle cell targeting. The first is a peptide sequence with enhanced *in vivo* skeletal and cardiac muscle binding, that was identified by screening a random phage display library (Samoylova and Smith, 1999). Muscle selectivity of the phage clone carrying this peptide was estimated to be in the range of 9- to 20-fold for skeletal and 5- to 9-fold for cardiac muscle (depending on control tissue) as compared to phage with no insert. However, it has not yet been shown whether or not this peptide can be used for *in vivo* targeting of conjugated compounds to muscle cells. The other molecule that has been described is an Fv part of a monoclonal antibody (mAb) that is selectively transported into skeletal muscle *in vivo* (Weisbart et al. 2003). Single chain Fv fragments of the murine mAb were injected into the tail veins of mice and 4 hours later the fragments were found in 20% of skeletal muscle cells, primarily localized in the nucleus. It was shown that the mAb binds to the protein myosin IIb in lysates of skeletal muscle cells, but it did not bind any protein in lysates of heart muscle cells. Therefore, this antibody might be useful for targeting to skeletal muscles, but not to the heart muscle.

Mannose-6-phosphate (M6P) residues are uniquely recognized by the two members of the P-type lectin family, the ∼ 46-kDa cation dependent mannose-6-phosphate receptor (CD-MPR) and the ∼ 300 kDa insulin-like growth factor II/mannose-6-phosphate receptor (IGF-II/MPR) (Dahms and Hancock, 2002). The P-type lectins play an essential role in the generation of functional lysosomes within the cells of higher eukaryotes, by directing newly synthesized lysosomal enzymes bearing the M6P signal to lysosomes. Lysosomal enzymes are synthesized by membrane bound ribosomes and translocated to the endoplasmic reticulum (ER), where the nascent proteins are glycosylated with high-mannose oligosaccharide chains. The mannose residues are then phosphorylated during further transit of the proteins through the ER-Golgi biosynthetic pathway, generating the M6P ligand used in targeting of the lysosomal enzymes to the lysosome via the M6P-receptors (Dahms and Hancock, 2002). At the cell surface the IGF-II/MPR, but not the CD-MPR, binds and internalizes a diverse population of M6P-containing proteins and is responsible for endocytosis of the majority of extracellular lysosomal enzymes (Ghosh et al. 2003; Hassan, 2003). The IGF-II/MPR is present in several human tissues such as kidney, liver, spleen and lung and also in heart and skeletal muscle (Funk et al. 1992; Wenk et al. 1991), and can therefore be used for targeting and uptake of M6P-containing compounds into the lysosomal compartment of muscle cells. The feasibility of such an approach has been demonstrated with the lysosomal enzyme α-glucosidase (GAA). First of all, it was shown that GAA isolated from bovine testis was endocytosed in a M6P-receptor dependent manner by cultured human skeletal muscle cells, obtained from muscle biopsies (Reuser et al. 1984). The uptake could completely be inhibited by M6P and by bovine testis β-galactosidase, a lysosomal enzyme bearing phosphorylated high-mannose-type sugar chains. These results show that M6P-receptors are present on the plasma membrane of skeletal muscle cells and engaged in the uptake of M6P-containing lysosomal enzymes. Also, when recombinant human GAA (rhGAA), produced in CHO-cells or mouse milk, was added to human GAA -/- fibroblasts in cell culture, the enzyme was internalized in a M6P-receptor dependent manner (Bijvoet et al. 1998; Martiniuk et al. 2000). Finally, after injection of rhGAA into GAA knockout mice, uptake into heart, skeletal muscles, legs and respiratory muscles, among which diaphragm, was demonstrated (Bijvoet et al. 1998; Martiniuk et al. 2000).

US 6,172,208 describes a conjugate comprising a glycoside, in particular mannose-6-phosphate, linked to an oligonucleotide.

### DISCLOSURE OF THE INVENTION

The present invention provides a novel method of delivering compounds into extra-lysosomal compartments, such as the cytoplasm, ER and the nucleus, of cells, in particular muscle cells. It was unexpectedly found that conjugates comprising a glycoside, such as the monosaccharide M6P, were able to deliver linked compounds into the nucleus of muscle cells, despite the prior art teaching that M6P is specifically targeted to the lysosomal compartment of cells.

In one embodiment of the invention glycoside-compound conjugates are provided. A "conjugate" as used herein refers to a ligand, such as a glycoside, which is chemically conjugated to a compound of interest. The ligand is able to bind to a specific receptor and thereby directs (or targets) the conjugate to this receptor. In one embodiment of the invention the ligand is capable of binding to an M6P receptor, preferably to IGF-II/MPR. Preferably the M6P receptor is of a muscle cell. The glycoside is preferably a mono-, di- or a higher order saccharide. In a preferred embodiment the saccharide is a M6P residue, although other saccharides with binding specificity for muscle cell receptors can be used. The conjugate may comprise one, two three or more glycosides. For example, the conjugate may comprise (M6P)2 or (M6P)4 or additional M6P residues. In case the conjugate comprises more than one glycoside it is preferred the terminal glycoside is an M6P.

Thus the invention relates to the use of a conjugate of a glycoside linked to an oligonucleotide selected from RNA, DNA, morpholino, 2'-O-methyl RNA, 2'-O-allyl RNA, Peptide Nucleic Acid (PNA) and Locked Nucleic Acid (LNA), wherein said glycoside is a ligand capable of binding to a mannose-6-phosphate receptor of a muscle cell for the preparation of a medicament for the treatment of a disease and wherein the delivery of said oligonucleotide is to an extra-lysosomal compartment of said muscle cell. Also the invention concerns a conjugate comprising a glycoside linked to an oligonucleotide, wherein said oligonucleotide is selected from RNA, DNA, morpholino, 2'-O-methyl RNA, or 2'-O-allyl RNA, Peptide Nucleic Acid (PNA) and Locked Nucleic Acid (LNA), said glycoside being a ligand capable of binding to a mannose-6-phosphate receptor of a muscle cell and said oligonucleotide comprising at least 20 nucleotides identical or complementary to a human dystrophin gene.

In an embodiment said oligonucleotide or derivative or mimic thereof is in antisense orientation. In an embodiment said oligonucleotide or derivative or mimic thereof comprises at least 20 nucleotides identical or complementary to a human dystrophin gene. In an embodiment said oligonucleotide or derivative or mimic thereof is selected from one of the following: morpholino, 2'-O-methyl RNA or 2'-O-allyl RNA.

In a preferred embodiment said mannose-6-phosphate receptor is an insulin-like growth factor II/mannose-6-phosphate receptor (IGF-II/MPR).

In an embodiment said glycoside of the conjugate of the invention is a mono-, di- or tri-saccharide, or any higher order saccharide, and wherein said saccharide comprises at least one mannose-6-phosphate residue. In a further embodiment said saccharide comprises at least two mannose-6-phosphate residues. In yet a further embodiment said di-, tri- or higher order saccharides are linked via (α1,2), (α1,3) or (α1,6) linkages. In yet another embodiment said glycoside is a bi-antennary or tri-antennary oligosaccharide comprising mono-, di- or tri-saccharides or any higher order saccharides, wherein said saccharides comprise at least one mannose-6-phosphate residue, preferably said saccharides comprise at least two mannose-6-phosphate residues.

In a further embodiment said compound of the conjugate of the invention is a growth factor, a vaccine, a vitamin, an antibody or a cationic entity to complex nucleic acids.

In yet a further embodiment said glycoside is linked to said compound via a labile spacer that can be cleaved intracellularly.

In another embodiment the invention relates to a method for producing a glycoside-compound conjugate, characterised by linking at least one glycoside comprising at least one mannose-6-phosphate residue with an oligonucleotide selected from any one of the following: DNA, RNA, PNA, LNA, morpholino, 2'-O-methyl RNA, or 2'-O-allyl RNA.

The M6P targeting system is meant for import into the lysosomal compartment of cells and GAA can only exert its effect in the lysosomes where it must, and does in a therapeutic setting, hydrolyse glycogen causing the disease.

The exon splicing process takes place in the nucleus and certainly not in the lysosomes where there is no RNA to be spliced. The surprising discovery we made is that M6P when coupled to an antisense molecule complementary to a splice site can also direct its cargo to the splicing machinery which is at a location distinctly different from the " well-known destination" normally used by M6P and its cargo (GAA). This unexpected discovery made it possible for us to use M6P to target muscle cells with bioactive compounds to various cellular compartments such as the nucleus (as an unexpected result, since the M6P targeting system is believed to direct cargo to the lysosomal compartment).

Thus the invention further concerns the use of any of the glycoside-compound conjugates of the invention to alter the sequence of an RNA or its precursors, to modify or modulate its composition and arrangement of its exons such that a protein can be made able to restore functionality of a cell to which it is delivered, in particular of muscle cells. In one aspect the glycoside-compound conjugates of the invention may be used to block or stimulate any RNA that can lead to improved performance of heart, respiratory or skeletal muscles with the aim to ameliorate the progression of certain diseases or impairments associated with e.g. ageing.

In another aspect the invention descrides a method for delivering a compound into the nucleus of cells comprising an insulin-like growth factor II/mannose-6-phosphate receptor (IGF-II/MPR), in which a glycoside-compound conjugate of the invention is contacted with said cells. In one aspect said contacting comprises injection into animal or human tissue. In another aspect said cells are muscle cells of a Duchenne Muscular Dystrophy (DMD) patient and said compound is an antisense oligonucleotide which causes exon skipping, in particular exon 46 skipping, and induces or restores the synthesis of dystrophin or variants thereof.

In a further aspect the invention describes a method for treating muscle diseases such as Beckers Muscular Dystrophy, spinal muscular atrophy (SMA), bethlem myopathy, myotubular myopathy, limb-girdle muscular dystrophy 2A and 2B, Miyoshi myopathy and merosin deficient muscular dystrophy, in which muscle cells of patients are contacted with a glycoside-compound conjugate of the invention.

Further the invention describes a method for inducing the synthesis or functioning of any RNA species in muscle cells, in which said cells are contacted with a glycoside-compound conjugate of the invention, whereby said compound inhibits or reduces the activity of RNAs or proteins repressing the synthesis or functioning of said RNA species.

Further the invention describes a method for inhibiting the synthesis or functioning of any RNA species in muscle cells which causes disease or predisposition of disease, which may be of viral or bacterial origin, in which said muscle cells are contacted with a glycoside-compound conjugate according to the invention, whereby said compound inhibits the synthesis or functioning of said RNA species.

Further the glycoside-compound conjugates of the invention may be of use to increase the body muscle mass of farm animals. For instance muscle cells may be targeted with a conjugate comprising a compound which is designed to increase muscle mass, such as for instance an oligonucleotide that inhibits myostatin production. Accordingly the invention also relates to such a method.

Further the invention describes a method for delivering a vaccine into muscle cells, in which muscle cells are contacted with a glycoside-compound conjugate according to the invention, wherein said compound is a vaccine.

Further the invention describes the use of a glycoside-compound conjugate according to the invention in the therapeutic treatment of muscle diseases. In particular the invention relates to the use of a glycoside-compound conjugate according to the invention for the preparation of a medicament. In an embodiment the invention relates to the use of a glycoside-compound conjugate according to the invention for the preparation of a medicament for the therapeutic treatment of muscle diseases.

In a further embodiment the glycoside-compound conjugate further comprises a marker. In an embodiment said marker is directly or indirectly detectable by visual, chemical or molecular methods. In an embodiment said marker is a fluorescent marker, a chemiluminescent marker, a radioactive marker, an enzymatic marker or molecular marker.

Further the invention describes a method for in vivo or in vitro diagnostic tests, in which a conjugate of the invention further comprising a marker is contacted with muscle cells and detecting directly or indirectly the presence or absence of said marker. Yet further the invention concerns a diagnostic detection kit comprising a conjugate of the invention further comprising a marker and optionally further comprising detection reagents

### EXAMPLES

### Example 1: Overview building blocks for synthesis of the glycoside-compounds

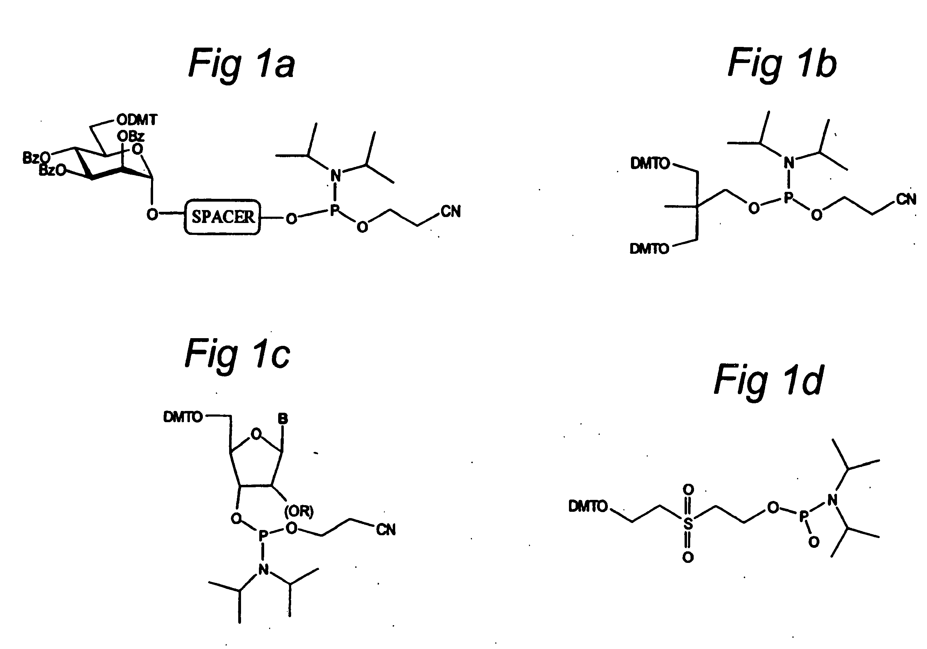

To be able to produce the glycoside-compounds, a multiple step synthesis was designed. All syntheses were performed using standards organic chemical synthesis procedures. The separate building blocks 1A and 1B (Fig 1A and 1B respectively) were synthesised, whereas the remaining blocks (Fig 1C and 1D) were purchased (Figure 1).

### Example 2: Assembly of building block 1

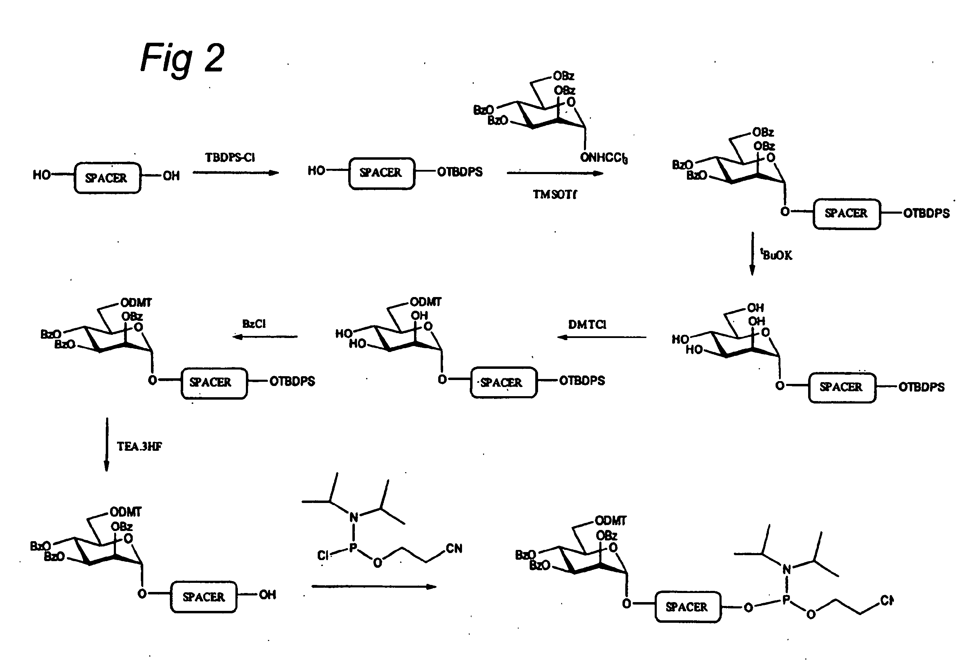

Building block 1 (Figure 2) is composed of the glycoside linked through a SPACER to a moiety X. SPACER is composed of a C4-, C5-, or C11-alkyl or tetrathylene glycol. Moiety X is composed of a phosphate, amide or disulfide bond.

### Example 3: Assembly of building block 2

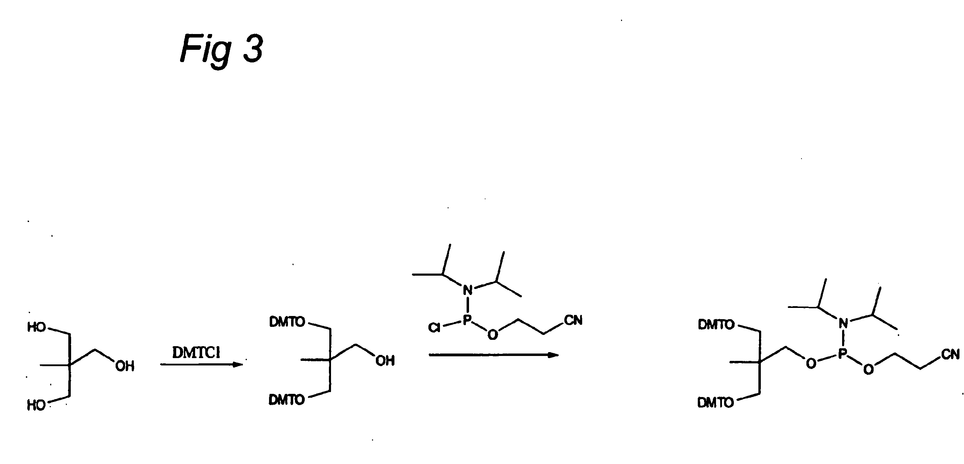

Building block 2 (Figure 3) is designed to connect Building block 1 to the compound, in example 4 to an oligonucleotide.

### Example 4: Assembly of the (man-6P)₂- en (man-6P)₄-oligonucleotides with C₄-, C₅-, and C₁₁-alkyl and tetraethylene glycol spacers

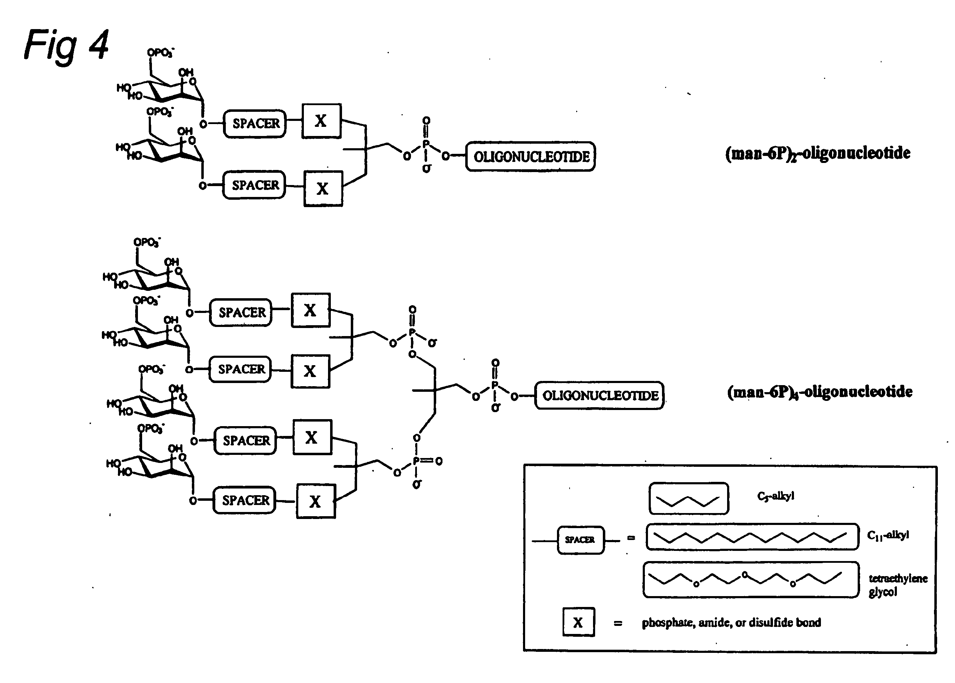

Using standard amidite solid phase synthesis the (man-6)ₓ-oligonucleotides were synthesized (Figure 4).

### REFERENCES

Aartsma-Rus A., Bremmer-Bout M., Janson A. A. M., den Dunnen J. T., van Ommen G. J. B. and van Deutekom J. C. T. (2002) Targeted exon skipping as a potential gene correction therapy for Duchenne muscular dystrophy. Neuromuscul. Disord. 12, S71-S77.
Bijvoet A. G. A., Kroos M. A., Pieper F. R., Van der Vliet M., de Boer H. A., Van der Ploeg A. T., Verbeet M. P. and Reuser A. J. (1998) Recombinant human acid α-glucosidase: high-level production in mouse milk, biochemical characteristics, correction of enzyme deficiency in GSDII KO mice. Hum. Mol. Genet. 7, 1815-1824.
Dahms N. M. and Hancock M. K. (2002) P-type lectins. Biochim. Biophys. Acta 1572, 317-340.
Funk B., Kessler U., Eisenmenger W., Hansmann A., Kolb H. J. and Kiess W. (1992) Expression of the insulin-like growth factor-II/mannose-6-phosphate receptor in multiple human tissues during fetal life and early infancy. J. Clin. Endocrinol. Metab. 75, 424-431.
Ghosh P., Dahms N. M. and Komfeld S. (2003) Mannose 6-phosphate receptors: new twists in the tale. Nat. Rev. Mol. Cell Biol. 4, 202-212.
Gollins H., McMahon J., Wells K. E. and Wells D. J. (2003) High-efficiency plasmid gene transfer into dystrophic muscle. Gene Ther. 10, 504-512.
Hassan A. B. (2003) Keys to the hidden treasures of the mannose 6-phosphate/insulin-like growth factor 2 receptor. Am. J. Pathol. 162, 3-6.
Herweijer H., Whitesell L. F., Buck J., Bates M. K., Budker V., Zhang G., Wolff J. A. and Wolff M. R. (2000) Retrograde coronary venous delivery of naked plasmid DNA. Mol. Ther. 1, S202.
Herweijer H. and Wolff J. A. (2003) Progress and prospects: naked DNA gene transfer and therapy. Gene Ther. 10, 453-458.
Lu Q. L., Liang H.-D., Partridge T. and Blomley M. J. K. (2003) Microbubble ultrasound improves the efficiency of gene transduction in skeletal muscle in vivo with reduced tissue damage. Gene Ther. 10, 396-405.
Martiniuk F., Chen A., Donnabella V., Arvanitopoulos E., Slonim A. E., Raben N., Plotz P. and Rom W. N. (2000) Correction of glycogen storage disease type II by enzyme replacement with a recombinant human acid maltase produced by over-expression in a CHO-DHFRneg cell line. Biochem. Biophys. Res. Commun. 276, 917-923.
Reuser A. J. J., Kroos M. A., Ponne N. J., Wolterman R. A., Loonen M. C. B., Busch H. F. M., Visser W. J. and Bolhuis P. A. (1984) Uptake and stability of human and bovine acid α-glucosidase in cultured fibroblasts and skeletal muscle cells from glycogenosis type II patients. Exp. Cell Res. 155, 178-189.
Samoylova T. I. and Smith B. F. (1999) Elucidation of muscle-binding peptides by phage display screening. Muscle Nerve 22, 460-466.
van Deutekom J. C. T., Bremmer-Bout M., Janson A. A. M., Ginjaar I. B., Baas F., den Dunnen J. T. and van Ommen G. J. B. (2001) Antisense-induced exon skipping restores dystrophin expression in DMD patient derived muscle cells. Hum. Mol. Genet. 10, 1547-1554.
Weisbart R. H., Yang F., Chan G., Wakelin R., Ferreri K., Zack D. J., Harrison B., Leinwand L. A. and Cole G. M. (2003) Cell type specific targeted intracellular delivery into muscle of a monoclonal antibody that binds myosin IIb. Mol. Immunol. 39, 783-789.
Wenk J., Hille A. and von Figura K. (1991) Quantitation of Mr 46000 and Mr 300000 mannose-6-phosphate receptors in human cells and tissues. Biochem. Int. 23, 723-731.
Zhang G., Budker V., Williams P., Subbotin V. and Wolff J. A. (2001) Efficient expression of naked DNA delivered intra-arterially to limb muscles of nonhuman primates. Hum. Gene Ther. 12, 427-438.

## Claims

1. Use of a conjugate of a glycoside linked to an oligonucleotide selected from RNA, DNA, morpholino, 2'-O-methyl RNA, 2'-O-allyl RNA, Peptide Nucleic Acid (PNA) and Locked Nucleic Acid (LNA), wherein said glycoside is a ligand capable of binding to a mannose-6-phosphate receptor of a muscle cell for the preparation of a medicament for the delivery of said oligonucleotide to an extra-lysosomal compartment of said muscle cell.

2. The use according to claim 1, wherein the medicament is for the treatment of a muscle disease.

3. The use according to claim 1 or 2, wherein the muscle disease is selected from Beckers Muscular Dystrophy, spinal muscular atrophy (SMA), bethlem myopathy, myotubular myopathy, limb-girdle muscular dystrophy 2A and 2B, Miyoshi myopathy and merosin deficient muscular dystrophy.

4. The use according to any one of claims 1-3, wherein said glycoside is a mono-, di- or tri-saccharide, or any higher order saccharide, and wherein said saccharide comprises at least one mannose-6-phosphate residue.

5. The use according to any one of claims 1-4, wherein said glycoside is a bi-antennary or tri-antennary oligosaccharide comprising mono-, di- or tri-saccharides or any higher order saccharides, wherein said saccharides comprise at least one mannose-6-phosphate residue.

6. The use according to any one of claims 1-5, wherein said glycoside is linked to said oligonucleotide via a labile spacer that can be cleaved intracellularly.

7. The use according to any one of claims 1-5, wherein said oligonucleotide is linked to said glycoside via a cationic entity to complex nucleic acids.

8. The use according to any one of claims 1-7, wherein said mannose-6-phosphate receptor is an insulin-like growth factor ll/mannose-6-phosphate receptor (IGF-II/MPR).

9. The use according to claim 8 wherein the delivery is into the nucleus of a muscle cell, wherein said muscle cell comprises an insulin-like growth factor II/mannose-6-phosphate receptor (TGF-II/MPR).

10. The use according to any one of claims 1-9, wherein said muscle cells are of a Duchenne Muscular Dystrophy (DMD) patient and wherein said oligonucleotide is an antisense oligonucleotide which causes exon skipping and induces or restores the synthesis of dystrophin or variants thereof.

11. The use according to any one of claims 1-10, wherein said oligonucleotide or derivative or mimic thereof comprises at least 20 nucleotides identical or complementary to a human dystrophin gene.

12. The use according to any one of claims 1-9, wherein the composition is for inducing the synthesis or function of any RNA species in muscle cells wherein said oligonucleotide of said conjugate inhibits or reduces the activity of RNAs or proteins thereby repressing the synthesis or functioning of said RNA species.

13. The use according to any one of claims 1-9, wherein the composition is for inhibiting the synthesis or function of any RNA species in muscle cells which causes disease or predisposition to a disease, wherein said oligonucleotide of said conj ugate inhibits the synthesis or function of said RNA species.

14. The use according to any one of claims 1-13 wherein said conjugate further comprises a marker.

15. The use according to claim 14 for in vivo or in vitro diagnostic tests comprising detecting directly or indirectly the presence or absence of said marker.

16. A conjugate comprising a glycoside linked to an oligonucleotide, wherein said oligonucleotide is selected from RNA, DNA, morpholino, 2'-O-methyl RNA, or 2'-O-avyl RNA, Peptide Nucleic Acid (PNA) and Locked Nucleic Acid (LNA), said glycoside being a ligand capable of binding to a mannose-6-phosphate receptor of a muscle cell and said oligonucleotide comprising at least 20 nucleotides identical or complementary to a human dystrophin gene.

17. The conjugate according to claim 16, wherein said glycoside is a mono-, di- or tri-saccharide, or any higher order saccharide, and wherein said saccharide comprises at least one manaose-6-phosphate residue.

18. The conjugate according to claim 16, wherein said glycoside is a bi-antennary or tri-antennary oligosaccharide comprising mono-, di- or tri-saccharides or any higher order saccharides, wherein said saccharides comprise at least one mannose-6-phosphate residue.

19. The conjugate according to any one of claims 16-18, wherein said glycoside is linked to said oligonucleotide via a labile spacer that can be cleaved intracellularly.

20. The conjugate according to any one of claims 16-19, said conjugate further comprising a marker.

## Patentansprüche

1. Verwendung eines Konjugats eines Glykosids, das an ein Oligonukleotid, ausgewählt aus RNA, DNA, Morpholin, 2'-O-Methyl-RNA, 2'-O-Allyl-RNA, Peptid-Nuklein-Säure (PNA) und verschlossene Nukleinsäure (LNA) gebunden ist, wobei das Glykosid ein Ligand ist, der in der Lage ist, an einen Mannose-6-Phosphat-Rezeptor einer Muskelzelle zu binden, zur Herstellung eines Arzneimittels für die Abgabe des Oligonukleotids an ein extralysosomales Kompartment der Muskelzelle.

2. Verwendung nach Anspruch 1, wobei das Arzneimittel zur Behandlung einer Muskelkrankheit vorgesehen ist.

3. Verwendung nach Anspruch 1 oder 2, wobei die Muskelkrankheit ausgewählt ist aus Becker-Muskeldystrophie, spinale Muskelatrophie (SMA), Bethlem-Myopathie, myotubulare Myopathie, Gliedergürtel-Muskeldystrophie 2A und 2B, Miyoshi-Myopathie und Merosinmangel-Muskeldystrophie.

4. Verwendung nach einem der Ansprüche 1 bis 3, wobei das Glykosid ein Mono-, Di- oder Tri-Saccharid oder ein beliebiges höhergradiges Saccharid ist, und wobei das Saccharid wenigstens einen Mannose-6-Phosphat-Rest umfasst.

5. Verwendung nach einem der Ansprüche 1 bis 4, wobei das Glykosid ein Zwei-Antennen oder Drei-Antennen-Oligosaccharid ist, das Mono-, Di- oder Tri-Saccharide oder beliebige höhergradige Saccharide umfasst, wobei die Saccharide wenigstens einen Mannose-6-Phosphat-Rest umfassen.

6. Verwendung nach einem der Ansprüche 1 bis 5, wobei das Glykosid an das Oligonukleotid über einen labilen Spacer gebunden ist, der intrazellulär gespalten werden kann.

7. Verwendung nach einem der Ansprüche 1 bis 5, wobei das Oligonukleotid an das Glykosid über einen Kationenrest an komplexe Nukleinsäuren gebunden ist.

8. Verwendung nach einem der Ansprüche 1 bis 7, wobei der Mannose-6-Phosphat-Rezeptor ein Insulin-ähnlicher Wachstumsfaktor II/Mannose-6-Phosphat-Rezeptor (IGF-II/MPR) ist.

9. Verwendung nach Anspruch 8, wobei die Abgabe in den Nukleus einer Muskelzelle erfolgt, wobei die Muskelzelle einen Insulin-ähnlichen Wachstumsfaktor II/Mannose-6-Phosphat-Rezeptor (IGF-II/MPR) umfasst.

10. Verwendung nach einem der vorhergehenden Ansprüche 1 bis 9, wobei die Muskelzellen von einem Duchenne-Muskeldystrophie (DND)-Patienten stammen und wobei das Oligonukleotid ein Antisinn-Oligonukleotid ist, das ein Exon-Springen bewirkt und die Synthese von Dystrophin oder Varianten davon induziert oder wieder herstellt.

11. Verwendung nach einem der Ansprüche 1 bis 10, wobei das Oligonukleotid oder das Derivat oder das Imitat davon wenigstens 20 Nukleotide umfasst, die identisch oder komplementär zu einem humanen Dystrohpien-Gen sind.

12. Verwendung nach einem der Ansprüche 1 bis 9, wobei die Zusammensetzung zur Induktion der Synthese oder Funktion einer beliebigen RNA-Spezies in Muskelzellen vorgesehen ist, wobei das Oligonukleotid des Konjugats die Aktivität von RNAs oder Proteinen inhibiert oder vermindert, wodurch die Synthese oder die Funktion der RNA-Spezies unterdrückt wird.

13. Verwendung nach einem der Ansprüche 1 bis 9, wobei die Zusammensetzung zur Inhibition der Synthese oder Funktion einer beliebigen RNA-Spezies in Muskelzellen vorgesehen ist, welche eine Krankheit oder Veranlagung für eine Krankheit bewirkt, wobei das Oligonukleotid des Konjugats die Synthese oder Funktion der RNA-Spezies inhibiert.

14. Verwendung nach einem der Ansprüche 1 bis 13, wobei das Konjugat ferner einen Marker umfasst.

15. Verwendung nach Anspruch 14, für in-vivo- oder in-vitro-Diagnosetests, welche das Vorliegen oder das Fehlen des Markers direkt oder indirekt detektieren umfasst.

16. Konjugat, umfassend ein Glykosid, das an ein Oligonukleotid gebunden ist, wobei das Oligonukleotid ausgewählt ist aus RNA, DNA, Morpholin, 2'-O-Methyl-RNA oder 2'-O-Allyl-RNA, Peptid-Nukleinsäure (PNA) und verschlossene Nukleinsäure (LNA), wobei das Glykosid ein Ligand ist, das in der Lage ist, sich an einen Mannose-6-Phosphat-Rezeptor einer Muskelzelle zu binden und wobei das Oligonukleotid wenigstens 20 Nukleotide umfasst, die identisch oder komplementär zu einem humanen Dystrophin-Gen sind.

17. Konjugat nach Anspruch 16, wobei das Glykosid ein Mono-, Di- oder Tri-Saccharid oder ein beliebiges höhergradiges Saccharid ist, und wobei das Saccharid wenigstens einen Mannose-6-Phosphat-Rest umfasst.

18. Konjugat nach Anspruch 16, wobei das Glykosid ein Zwei-Antennen- oder Drei-Antennen-Oligosaccharid ist, das Mono-, Di- oder Tri-Saccharide oder beliebige höhergradige Saccharide umfasst, wobei die Saccharide wenigstens einen Mannose-6-Phosphat-Rest umfassen.

19. Konjugat nach einem der Ansprüche 16 bis 18, wobei das Glykosid an das Oligonukleotid über einen labilen Spacer gebunden ist, der intrazellulär gespalten werden kann.

20. Konjugat nach einem der Ansprüche 16 bis 19, wobei das Konjugat ferner einen Marker umfasst.

## Revendications

1. Utilisation d'un conjugué d'un glycoside lié à un oligonucléotide choisi parmi les ARN, ADN, morpholino, 2'-O-méthyl-ARN, 2'-O-allyl-ARN, Acide Nucléique Peptidique (PNA) et Acide Nucléique verrouillé (LNA), où ledit glycoside est un ligand capable de se lier à un récepteur de mannose-6-phosphate d'une cellule musculaire, pour la préparation d'un médicament destiné à la libération dudit oligonucléotide dans un compartiment extra-lysosomial de ladite cellule musculaire.

2. Utilisation selon la revendication 1, où le médicament est destiné au traitement d'une maladie musculaire.

3. Utilisation selon la revendication 1 ou 2, où la maladie musculaire est choisie parmi la myopathie pseudo-hypertrophique de Becker, l'atrophie musculaire spinale (SMA), la myopathie de Bethlem, la myopathie myotubulaire, la dystrophie musculaire des ceintures 2A et 2B, la myopathie de Miyoshi et la dystrophie musculaire déficiente en mérosine.

4. Utilisation selon l'une quelconque des revendications 1-3, où ledit glycoside est un mono-, di- ou tri-saccharide, ou n'importe quel saccharide d'ordre supérieur, et où ledit saccharide comprend au moins un résidu mannose-6-phosphate.

5. Utilisation selon l'une quelconque des revendications 1-4, où ledit glycoside est un oligosaccharide bi-antennaire ou tri-antennaire comprenant des mono-, di- ou tri-saccharides ou n'importe quels saccharides d'ordre supérieur, lesdits saccharides comprenant au moins un résidu mannose-6-phosphate.

6. Utilisation selon l'une quelconque des revendications 1-5, où ledit glycoside est lié audit oligonucléotide par l'intermédiaire d'un espaceur labile qui peut être clivé au niveau cellulaire.

7. Utilisation selon l'une quelconque des revendications 1-5, où ledit oligonucléotide est lié audit glycoside par l'intermédiaire d'une entité cationique à des acides nucléiques complexes.

8. Utilisation selon l'une quelconque des revendications 1-7, où ledit récepteur de mannose-6-phosphate est un facteur de croissance analogue à l'insuline II/récepteur de mannose-6-phosphate (IGF-II/MPR).

9. Utilisation selon la revendication 8, où la libération se fait dans le noyau d'une cellule musculaire, ladite cellule musculaire comprenant un facteur de croissance analogue à l'insuline II/récepteur de mannose-6-phosphate (IGF-II/MPR).

10. Utilisation selon l'une quelconque des revendications 1-9, où lesdites cellules musculaires sont celles d'un patient atteint de dystrophie musculaire de Duchenne (DMD) et où ledit oligonucléotide est un oligonucléotide antisens qui provoque une omission d'exon et induit ou restaure la synthèse de la dystrophine ou de ses variants.

11. Utilisation selon l'une quelconque des revendications 1-10, où ledit oligonucléotide ou l'un de ses dérivés ou composés mimétiques comprend au moins 20 nucléotides identiques ou complémentaires à un gène de dystrophine humain.

12. Utilisation selon l'une quelconque des revendications 1-9, où la composition sert à induire la synthèse ou la fonction d'une espèce d'ARN quelconque dans les cellules musculaires dans lesquelles ledit oligonucléotide dudit conjugué inhibe ou réduit l'activité des ARN ou des protéines, réprimant ainsi la synthèse ou la fonction de ladite espèce d'ARN.

13. Utilisation selon l'une quelconque des revendications 1-9, où la composition sert à inhiber la synthèse ou la fonction d'une espèce d'ARN quelconque dans les cellules musculaires, qui provoque une maladie ou une prédisposition à une maladie, où ledit oligonucléotide dudit conjugué inhibe la synthèse ou la fonction de ladite espèce d'ARN.

14. Utilisation selon l'une quelconque des revendications 1-13, où ledit conjugué comprend en outre un marqueur.

15. Utilisation selon la revendication 14 pour des tests de diagnostic in vivo ou in vitro, comprenant la détection directe ou indirecte de la présence ou de l'absence dudit marqueur.

16. Conjugué comprenant un glycoside lié à un oligonucléotide, dans lequel ledit oligonucléotide est choisi parmi les ARN, ADN, morpholino, 2'-O-méthyl-ARN, ou 2'-O-allyl-ARN, Acide Nucléique Peptidique (PNA) et Acide Nucléique verrouillé (LNA), ledit glycoside étant un ligand capable de se lier à un récepteur de mannose-6-phosphate d'une cellule musculaire et ledit oligonucléotide comprenant au moins 20 nucléotides identiques ou complémentaires à un gène de dystrophine humain.

17. Conjugué selon la revendication 16, dans lequel ledit glycoside est un mono-, di- ou tri-saccharide, ou n'importe quel saccharide d'ordre supérieur, et dans lequel ledit saccharide comprend au moins un résidu mannose-6-phosphate.

18. Conjugué selon la revendication 16, dans lequel ledit glycoside est un oligosaccharide bi-antennaire ou tri-antennaire comprenant des mono-, di- ou tri-saccharides ou n'importe quels saccharides d'ordre supérieur, lesdits saccharides comprenant au moins un résidu mannose-6-phosphate.

19. Conjugué selon l'une quelconque des revendications 16-18, dans lequel ledit glycoside est lié audit oligonucléotide par l'intermédiaire d'un espaceur habile qui peut être clivé au niveau intracellulaire.

20. Conjugué selon l'une quelconque des revendications 16-19, ledit conjugué comprenant en outre un marqueur.
